# EUROPEAN PATENT APPLICATION

(11) **EP 3 130 351 A1**
(43) Date of publication of application: **15.02.2017**
(21) Application number: 14898405.7
(22) Date of filing: 29.07.2014
(51) Int. Cl.: A61K 41/00, A61P 35/00

(54) **A METHOD FOR TREATING CANCER BASED ON METALLOFULLERENE MONOCRYSTALLINE NANOPARTICLES THAT SPECIFICALLY DISRUPT TUMOR BLOOD VESSELS**

(71) Applicant: Beijing Fullcan Bio-technology Co., Ltd., Beijing 100085 (CN)
(72) Inventor: WANG, Chunru, Beijing 100080 (CN); ZHEN, Mingming, Beijing 100080 (CN); SHU, Chunying, Beijing 100080 (CN); WANG, Taishan, Beijing 100080 (CN); LI, Jie, Beijing 100080 (CN); ZHANG, Guoqiang, Beijing 100080 (CN)
(74) Representative: ABG Patentes, S.L.
(86) International application number: PCT/CN2014/000722
(87) International publication number: WO 2016/015172

(57) **Abstract**

The present invention discloses use of metallofullerene monocrystalline nanoparticles for the preparation of tumor vascular disrupting agents. The monocrystalline nanoparticles are water-soluble metallofullerene nanoparticles with negative charges on their surfaces, the particle sizes thereof range from 50 to 250 nanometers; moreover, the nanomaterials are able to absorb outside radiation energy, and transform it into heat energy, the volumes thereof will rapidly expand when temperature reached phase transformation point. When treatment, metallofullerene monocrystalline nanoparticles are administrated to a tumor-bearing organism via injection; the metallofullerene monocrystalline nanoparticles reach tumor sites via blood circulation, and retain at the sites of pores and defects of tumors; the monocrystalline nanoparticles of metallofullerene accumulate heat and the temperature increases under the role of outside radiation energy, the volumes sharply expand when the temperature exceeds critical point of phase transition thereof, thereby to cause changes in the morphologies, structures or functions of endothelium cells of tumor vessels, further to starve the tumor cells.

## Description

### Technical Field

The present invention belongs to the medical fields, and particularly relates to a method for the treatment of cancers based on metallofullerene monocrystalline nanoparticles specifically disrupting tumor vessels.

### Background Art

Both morbidity and mortality of cancers show a rising trend in recent years, and the newly added cancer patients are as many as 15 million annually around the world, seriously threatening human health. Traditionally, three major tumor therapies, that is, surgery, radiotherapy and chemotherapy, all have some limitations, causing that cancers become one of the leading reasons of human death. In recent years, with the development of science and technology, some new physical, chemical and biological methods, such as thermotherapy, radiofrequency ablation therapy, photodynamic therapy, and immunotherapy etc., occurred for treating tumors.

In 1971, Folkman et al. proposed that the growth and metastasis of solid tumors rely on tumor neovascularization, and further proposed a new therapy of cancers, which targets tumor angiogenesis, inhibits or disrupts tumor angiogenesis by tumor angiogenesis inhibitors, and cuts off the nutrition sources of tumors to "starve" tumors.

Vessels are the foundation of tumor reproduction, growth, development and reparation, and the great majority of malignant solid tumors such as ovarian cancer, liver cancer, cervical cancer and breast cancer are all vessel-dependent tumors. Tumor vessels are paraplastic vessels, which have great differences in terms of both structure and function, compared to normal vessels. A normal vessel generally consists of three membranes: intima, mesolamella and adventitia, but there is only one layer of thin intima in the structure of a tumor vessel. Furthermore, since the endothelium gap is bigger and the structure is incomplete, the tumor vessels contain a large number of nanosized micropores, through which the plasma can penetrate out. Furthermore, the blood transportation at tumors is abundant, so tumor vessels also have the characteristics of more blood flow and higher blood pressure which is triple of the normal vessel pressure. Based on above analysis of the differences between normal vessels and tumor vessels, a method that is directed to the characteristics of tumor vessels and specifically disrupts the tumor vessels can be designed to treat tumors.

At present, the researches directed to tumor neovessels have two major directions: angiogenesis inhibitors (AIs) and vascular disrupting agents (VDAs). VDAs refer to one kind of drug which can selectively disrupt malignant tumor neovessels, thereby to achieve the purpose of treating cancers. This concept was initially proposed by Denekamp in the 1980s. The proposal of developing VDAs was initially derived from the inspiration obtained from the experimental research in which tumor vessels were clogged by physical means to inhibit tumor growth, and was gradually developed into one kind of drug which achieves a similar curative effect with pharmacological functions to that via physical means. The mechanism of effect is to selectively bind to the malignant tumor neovessels, to directly or indirectly clog or damage the tumor neovessels, causing avascular necrosis of the tumor cells, thereby to play a role in treating the malignant tumors, based on the pathophysiologic differences between malignant tumor neovessels and normal vessels. However, the clinical trials of recently developed small molecule VDAs all reported adverse cardiovascular reactions, such as hypertension, tachycardia, bradyarrhythmia, atrial fibrillation, and myocardial infarction etc., and these adverse reactions limit their widely clinical uses.

### Disclosure of the Invention

One of the purposes of the present invention is to provide a new use of metallofullerene monocrystalline nanoparticles.

The new use of the metallofullerene monocrystalline nanoparticles provided by the present invention is its use for the preparation of tumor vascular disrupting agents.

Metallofullerene is one kind of new endohedral fullerene (also called endohedral metallofullerene) which is formed by embedding metal atoms into a fullerene carbon cage, and the same carbon cage can be embedded with various morphological metal atoms, such as monometal atoms, homonuclear or heteronuclear bimetal clusters, etc.

The metallofullerene types used in the present invention mainly contain: M@C₂ₙ, M₂@C₂ₙ, MA@C₂ₙ, M₃N@C₂ₙ, M₂C_{2@}C₂ₙ, M₂S@C₂ₙ, M₂O@C₂ₙ, and MₓA₃₋ₓN@C₂ₙ; wherein M and A both represent metal elements, M and A both are selected from any one of Sc, Y, and lanthanide metal elements (La-Lu), 30 ≤ n ≤ 60; and 0 ≤ x ≤ 3.

The metallofullerene used by the present invention not only includes paramagnetic metallofullerene, but also includes nonparamagnetic metallofullerene.

Wherein, the metallofullerene monocrystalline nanoparticles are water-soluble metallofullerene nanoparticles with negative charges on their surfaces; the particle sizes of the monocrystalline nanoparticles range from 50 to 250 nanometers; the monocrystalline nanoparticles have rigidity (not easy to deform), making them get stuck at the pores when they are passing through the vessel walls of the tumors; moreover, the nanoparticles are able to absorb outside radiation energy, and transform it into heat energy, resulting in increase in the inside accumulation temperature. When the temperature reaches phase transition temperature, the volumes of the monocrystalline nanoparticles radically expand, thereby to cause changes in the morphologies, structures or functions of the endothelium cells of the tumor vessels, achieving the purpose of blocking or disrupting tumor vessels.

The approach for the changes in the morphologies and/or structures of the metallofullerene monocrystalline nanoparticles is as follows: according to the differences of the types, sizes and surface-modified chemical groups of metallofullerene monocrystalline particles, the phase transition temperatures can be adjustable between 40-90°C, and when the accumulation temperature exceeds the phase transition temperature point of the material and the phase transition occurs, the volumes of the particles rapidly expand. If such metallofullerene nanoparticles are just located at the tumor vessels, phase transition thereof will disrupt vascular endothelium cells of the tumor, to cause their apoptosis or falling off from basilemma, so as to damage the tumor vessels and starve the cancer cells, achieving the effect of efficiently treating tumors.

The water-soluble metallofullerene nanoparticles with negative charges on their surfaces described in the present invention are obtained by water solubilization modifying metallofullerene monocrystalline nanoparticles, and guaranteeing the surface with electronegativity.

Certainly, in order to simplify the modification steps, the electronegativity modification for metallofullerene monocrystalline can be achieved synchronously by selecting a suitable water solubilization modification method.

The metallofullerene monocrystalline nanoparticles used by the present invention need to possess water-solubility, can be injected into the organism via veins, and transported to the tumor vessel along with the blood circulation to function therein. In order to realize the above objectives, the water solubilization modification for the metallofullerene monocrystalline nanoparticles is needed. There exist metallofullerene molecules in the monocrystalline form inside modified metallofullerene monocrystalline nanoparticles, while the exterior is modified with several water-soluble functional groups. These chemical functional groups contain one or more hydrophilic groups, e.g., hydroxyl, carboxyl, sulfhydryl, or amino etc., or the combination thereof, causing that the metallofullerene monocrystalline nanoparticles are water-soluble; or metallofullerene or the derivative crystal thereof is packaged directly by hydrophilic small biological molecules, such as amino acids, peptide chains etc.; metallofullerene or the derivative crystalline particle thereof also can be loaded with the help of carrier materials having biocompatibility, such as cladding by lipidosomes, cell membrane carriers etc.; and further, water-soluble supramolecular system can be formed by self-assembly, etc. The modification methods described above all can be performed according to the methods disclosed by the prior art.

The metallofullerene monocrystalline nanoparticles used by the present invention should carry with a certain electronegativity. The electronegativity modification methods include directly introducing electronegativity functional groups, such as hydroxyl, carboxyl and amino acid etc. onto the metallofullerene crystal surface by covalent bonds, and also non-covalently cladding by the carrier with electronegativity, such as hydroxylated silicon dioxide layers, carboxylated carbon films and peptide chains etc. They make the material difficult to aggregate in blood and no absorption onto the walls of normal vessels, and even if collision occurs, they can be rapidly bounced off, so that the damages to normal cells or biological tissues can be decreased when fullerene monocrystalline nanoparticles are phase changing.

The water-soluble metallofullerene monocrystalline nanoparticles with negative charges on their surfaces described in the present invention typically have the sizes of 50-250 nm in aqueous solution, and the monocrystalline nanoparticles have a certain rigidity (not easy to deform), making the nanoparticles difficult to pass through the nanopores between the endothelium cells of tumor vessels, but still able to be tightly attached onto the pore sites of the vessel walls via the differences between internal and external pressures of the vessels, to facilitate to more effectively destroy the endothelium cells of tumor vessels when the phase transition of nanoparticles causes the change in morphology and structure of the material.

In order to obtain the water-soluble metallofullerene monocrystalline nanoparticles with negative charges on their surfaces having rigid structures, the most effective approach is to prepare them by solid-liquid reaction under alkaline condition.

Such metallofullerene nanomaterials (i.e., water-soluble metallofullerene monocrystalline nanoparticles with negative charges on their surfaces) can absorb outside radiation energy, and rapidly accumulate energy inside the monocrystalline particles, and when the temperature exceeds the phase transition temperature point (40-90 °C) of such material, the volume of the material rapidly expands; and the used outside energy source mainly include: radiofrequency, microwave, infrared laser and x-ray etc.

The energy source of outside radiation described by the present invention mainly includes radiofrequency (also called radio wave), microwave, infrared light, visible light, laser, x-ray and alternating magnetic field etc., as well as any combination thereof. The form of energy is ultrashort pulse radiation as far as possible, for example, laser pulses, electromagnetic pulses (including radiofrequency pulses) etc., to facilitate the radiation energy absorption of the metallofullerene monocrystalline nanoparticles, such that the temperature rapidly rises, the phase transition occurs, and the energy is released. The pulse energy sources can simultaneously decrease the damage to the organism.

The metallofullerene monocrystalline nanoparticles applicable to the present invention can particularly be paramagnetic metallofullerene monocrystalline nanoparticles modified with polyhydroxy, such as Gd@C₈₂(OH)ₓ and Gd@C₆₀(OH)ₓ, further can be carboxyl derivatives of metallofullerene, also can have several derivatization groups such as Lu₃N@C₈₀(OH)ₓ(NH₂)_{y} or Gd@C₈₂(OH)ₓ(COOH)_{y} etc, wherein the x and y both represent integers between 10-30, generally even numbers.

The second purpose of the present invention is to provide a drug kit for treating tumors.

The drug kit provided by the present invention consists of metallofullerene monocrystalline nanoparticles described above and devices providing radiation energy source matched therewith.

The drug kit can particularly be nonparamagnetic metallofullerene monocrystalline nanoparticles plus pulse laser, or paramagnetic metallofullerene monocrystalline nanoclusters plus radiofrequency. When the endohedral metal in metallofullerene is a metal with great atomic number (e.g., La series metals), the drug kit also can be metallofullerene monocrystalline nanoparticles plus x-ray.

With respect to the energy source described above, those skilled in the art can make a reasonable selection according to the teaching of the prior art, and in the light of the absorption natures of the main materials for forming the nanomaterials towards different radiation energy.

The third purpose of the present invention is to provide a method for the treatment of tumors based on specific destruction of metallofullerene monocrystalline nanoparticles on tumor vessels.

The method for the treatment of tumors provided by the present invention includes the following steps:
1) Administrating effective dose of the water-soluble monocrystalline nanoparticles of the metallofullerene to a tumor-bearing organism in need of treatment via injection; and
2) Irradiating tumor sites of the tumor-bearing organism using the radiation energy source matched with the metallofullerene monocrystalline nanoparticles.

The irradiation time described by the present invention is to irradiate for a period of time, for example 10 min-2 h, at 0-1 h after injecting nanomaterials.

The "effective dose" described in the present invention refers to an enough amount to effectively transfer the active ingredients used for treating cancers when fullerene monocrystalline nanoparticles are administrated to the organism by the method of the present invention.

The organism described in the present invention refers to a mammal including a human being.

The preferred injection manner of the above method for the treatment of tumors is intravenous injection, in which the drug directly functions in blood, without permeation, the used dose of the drug is small, and the curative effect is high.

Under the role of radiofrequency with stronger penetrability, this treatment method not only can treat the tumors around the surfaces of the organism, but also can treat the tumors of the deep organs or tissues of the organism. Due to the special defects of physical therapy specific to tumor vessels, tumor types treated have wide-adaptability, the method is applicable to all solid tumors, including and not limited to common malignant tumor types, such as liver cancer, lung cancer, colorectal cancer, renal cancer, pancreatic cancer, bone cancer, breast cancer, ovarian cancer, prostate cancer, esophagus cancer, gastric cancer, oral cancer, nasal cancer, laryngeal cancer, liver cancer, bile duct cancer, cervical cancer, uterus cancer, testicular cancer, meningioma, skin cancer, melanoma, and sarcoma etc.

The treatment method described by the present invention has very high specificity for tumor vessels, and is not limited by the microenvironments of tumor tissues.

The metallofullerene monocrystalline nanoparticles used by the present invention do not have toxic and side effects on normal biological tissues while treating tumors, and have extremely high biosafety, which mainly reflects in:
1) The metal ions contained in metallofullerene are protected by fullerene shell, and will not leak within the organism. ¹³¹I labeled experiments prove that they can normally metabolize within the organism, most of metallofullerenes can be metabolized out of the body after 48 hours, and the residual metallofullerenes within the body are lower than the limit of detection after 7 days, so they can not be retained within the body and not bring long-term toxic and side effects.
2) Electronegative metallofullerene derivatives are difficult to be absorbed onto normal vessels and cell membranes, but directly act on tumor vessels via blood circulation, so they can function under low concentration of the drugs, which avoids the damage to the body.

In addition, the metallofullerene monocrystalline nanoparticles employed by the present invention further have the following functions:
1. Gadolinium metallofullerene can be used as a magnetic resonance contrast agent, and it can realize the real time observation of the morphological changes of tumors, so as to evaluate the therapeutic effects of metallofullerene nanomaterials on tumors. As for water-soluble fullerene derivatives modified with amino groups on their surfaces, the therapeutic effects on tumors also can be detected by fluorescence.
2. Metallofullerene derivatives have the characteristic of efficient free radical scavenging, and they not only do not damage normal tissues, but also can further protect normal cells.
3. Metallofullerene has dual functions of blocking tumor vessels under an outside radiation and inhibiting the growth of tumor vessels simultaneously.

In addition, the concentrations of the metallofullerene monocrystalline nanoparticles used by the present invention are 0.01-20 mmol/L in aqueous solution, the concentrations used in cell experiments are 0.01-1 mmol/L, and the results demonstrate that this drug does not have significant cytotoxicity; the drug concentrations used in animal experiments are 1-20 mmol/L, and after intravenous injection, mice do not have significant adverse reactions.

When used, the aqueous solution of metallofullerene monocrystalline nanoparticles with the concentration of 1-20 mmol/L is injected into organism via veins, and after blood circulation, they can be specifically and selectively retained at the pores of tumor vessels. 5-30 minutes after injection, outside radiation source (for example, radiofrequency) is applied onto the tumor sites, and the phase transition of metallofullerene nanomaterials occurs under the role of radiofrequency and their volumes rapidly expand, resulting in the endothelium cells of tumor vessels damaged, which facilitates cell apoptosis, thereby to expose the basilemma and collapse vasculatures inside solid tumors, resulting in the tumors ischemia and a wide range of necrosis.

Metallofullerene monocrystalline nanoparticles treat tumors mainly by the following mechanisms:
1. After intravenously injected, the water-soluble metallofullerene monocrystalline nanoparticles can be retained at the pores of tumor vessel endothelium cells or the defects of vessels via blood circulation, but are not accumulated in normal vessels. Since the difference between internal and external pressures of tumor vessels is great, the metallofullerene monocrystalline nanoparticles located at the pores are tightly embedded between endothelium cells of vessels.
2. Metallofullerene monocrystalline nanoparticles absorb energy to produce heat under the role of radiofrequency or laser, and the heat can be effectively accumulated inside the material. When the accumulation temperature exceeds the phase transition temperature point (40-90 °C) of the material, the volume thereof rapidly expands, which directly and quickly induces endothelium cell apoptosis of the tumor vessels, to expose the basilemma, followed by blood leakage of vessels, which cuts off the nutrition supplies to tumor tissues, to achieve the purpose of treating tumors.
3. Damaged tumor tissues are present inside the organism, and the immunoreactions stimulating the body further produce anti-tumor effect.
4. The special nature of metallofullerene in term of immunoreaction is utilized to further enhance the immune responses of the body, for example by adjusting the secretion of cytokines and facilitating the maturation of dendritic cells, etc.
5. Metallofullerene can block vessels by physical processes under the role of outside energy, and meanwhile it can also play a role in inhibiting the growth of tumor vessels per se.

Cell experiments verify that the metallofullerene monocrystalline nanoparticles described by the present invention do not have a significant killing effect on normal cells, this is because the surface electronegativity of such metallofullerene monocrystalline nanoparticles makes them difficult to aggregate in blood and not be absorbed by normal vessels, meanwhile the materials themselves have a function of protecting cells and can effectively avoid the damages to normal cells and tissues in the body.

The tumor treatment effects of water-soluble Gd@C₈₂ monocrystalline nanoparticles modified with hydroxyl on tumor-bearing mice (the tumor diameter is around 5 mm) under the role of radiofrequency are investigated. The in vivo magnetic resonance imaging experiments of experimental animals show that 4 hours after intravenously injecting into the bodies of tumor-bearing mice, significant changes of tumor sites can be observed; after 6 hours, the blood stasis phenomena occur at tumor sites; and the tumors which are internally hollow and have escharosis on surfaces are observed within 24 hours, but normal biological tissues, the organs such as liver, spleen, and kidney etc. remain intact. The imaging and anatomical results of animal experiments on other organs of tumor-bearing mice show that metallofullerene nanomaterials do not cause significant toxic and side effects on other organs, and can normally metabolize in the body.

The present invention provides a method for realizing efficient and quick treatment of malignant tumors utilizing metallofullerene monocrystalline nanoparticles to specifically destroy tumor vessels under the role of outside radiation energy (radiofrequency, microwave, infrared laser and x-ray etc.). Such an treatment process includes four stages, that is, (1) the paramagnetic metallofullerene is constructed to the rigid monocrystalline nanoparticles sized to 50-250 nanometers and modified with water-soluble groups on their surfaces, which are dissolved in ultrapure water and injected into veins of the organism; (2) metallofullerene monocrystalline nanoparticles reach tumor sites via blood circulation, and since a large number of pores and defects exist in the tumor vessels, suitable sized metallofullerene are retained at the sites of tumor pores and defects by blood pressure differences; (3) under the role of outside radiation energy, metallofullerene monocrystalline nanoparticles accumulate heat, and when the accumulation temperature exceeds the phase transition temperature point (40-90 °C) of the material, the volume of the material rapidly expands; (4) due to the quick changes of volumes caused by the phase transition, the metallofullerene monocrystalline nanoparticles can damage endothelium cells of the tumor vessels, and cause their apoptosis, resulting in the vessels rupture, which cuts off the nutrition supplies to tumor tissues, to finally achieve the effect of "starving" tumors. This method for the treatment of tumors has a therapeutic effect on many tumors, without performing a minimally invasive surgery, and effectively avoids the metastasis of tumors by cutting off the tumor vessels, so it is a broad-spectrum, quick, safe, and noninvasive efficient method for the treatment of tumors.

### Description of the Drawings

Figure 1 is a schematic view showing that metallofullerene monocrystalline nanoparticles specifically disrupt endothelium cells of tumor vessels; black particles in the figure represent the nanomaterials meeting the above natures; green particles represent the nanomaterials not meeting the above conditions; and the purple portions represent that endothelium cells of tumor vessels are damaged after the effect of nanomaterials.
Figure 2 is a photo of transmission electron microscope of gadolinium metallofullerene (Gd@C₈₂) monocrystalline nanoparticles modified with hydroxyl.
Figure 3 is a photo in which aqueous solution of the water-soluble Gd@C₈₂ monocrystalline nanoparticles produces a large number of bubbles under the irradiation of 13.5 MHz of radiofrequency.
Figure 4 are a series of photos of magnetic resonance imaging and state change of the tumor at each time point after the water-soluble Gd@C₈₂ monocrystalline nanoparticles are injected into the body of tumor-bearing mice via caudal vein followed by radiofrequency treatment (yellow arrows in the figure indicate tumor sites).
Figure 5 is a series of photos of therapeutic effect on the tumor after using water-soluble Gd@C₈₂ monocrystalline nanoparticles followed by radiofrequency treatment.
Figure 6 is a photo of environmental scanning electron microscope of tumor vessels without using water-soluble Gd@C₈₂ monocrystalline nanoparticles but with separate radiofrequency effect.
Figure 7 is a photo of environmental scanning electron microscope of tumor vessels subjected to the treatments of water-soluble Gd@C₈₂ monocrystalline nanoparticles and radiofrequency (yellow arrows in the figure are damaged sites of tumor vessels).
Figure 8 is a metabolic distribution of ¹³¹I labeled water-soluble Gd@C₈₂ monocrystalline nanoparticles in the bodies of tumor-bearing mice.

### Best Modes of the Invention

The present invention is illustrated with reference to particular examples below, but the present invention is not limited therein.

The experimental methods described in the below examples, unless expressly specified, all are conventional methods; and the reagents and materials, unless expressly specified, all can be obtained commercially.

The "metallofullerene monocrystalline nanoparticles" employed in the examples described below particularly are water-soluble gadolinium metallofullerene monocrystalline nanoparticles modified with hydroxyl (Gd@C₈₂).

The specific preparation method is as follows: oxidation reaction of the Gd@C₈₂ nanopower (Reference: Carbon, 2013, 65, 175) is performed under the role of hydrogen peroxide (H₂O₂ solution of 30% mass concentration), and Gd@C₈₂ water-soluble metallofullerene monocrystalline nanoparticles modified with hydroxyl outside the cage are obtained. Such nanoparticles are rigid structures, and since their surfaces are modified with a large number of hydroxyl groups, such rigid monocrystalline nanoparticles are charged with negative charges on their surfaces.

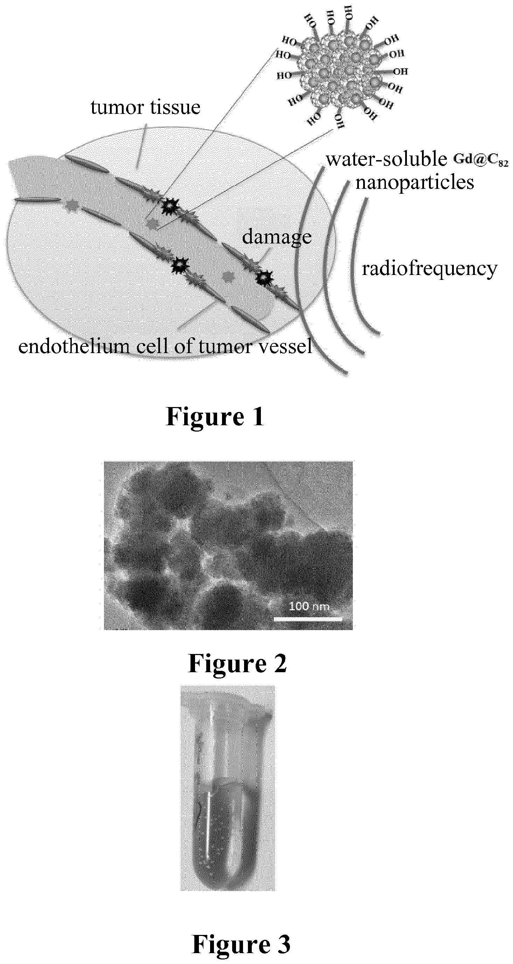

Figure 2 is a transmission photo of the water-soluble Gd@C₈₂ monocrystalline nanoparticles described above, the sizes of their particles are around 100 nm.

### Example 1: the experiment in which water-soluble Gd@C₈₂ monocrystalline nanoparticles produce bubbles under the role of radiofrequency

As seen in Figure 3, water-soluble Gd@C₈₂ monocrystalline nanoparticles were observed to continually produce a large number of bubbles in the solution during the process of radiofrequency effect (under the irradiation of 13.5 MHz of radiofrequency). This is because the water-soluble Gd@C₈₂ monocrystalline nanoparticles internally accumulate energy under the role of radiofrequency, and when accumulation temperature exceeds the phase transition temperature point (40-90 °C) of the material, the volume of the material rapidly expands, and bubbles are produced.

### Example 2: The verification of tumor treatment and effect of water-soluble Gd@C₈₂ monocrystalline nanoparticles under the role of radiofrequency

### 1) Establishing animal models of tumor-bearing mice:

Ascites was extracted from the mice inoculated with H22 liver cancer cell strains in enterocoelia thereof, the supernate was removed by centrifugation and cells were counted, and each mouse was subcutaneously inoculated with 50 µL of cell suspension having a cell concentration of 10⁷/mL in its right thigh. After 5-7 days' growth, the experiment was preformed when the tumor size is around 5 mm.

### 2) The magnetic resonance imaging and tumor treatment experiments of experimental living animals:

150 µL aqueous solution of water-soluble Gd@C₈₂ monocrystalline nanoparticles (the concentration is 10 mmol/L) was injected into the body of a tumor-bearing mouse via caudal vein. The radiofrequency pulses were applied for 1 h under a magnetic field (temperature: 37°C, frequency: 200 MHz, and band width: 500 kHz), and the imaging conditions of tumor tissues, kidney and liver were taken at each time point (before injection, and 30 min, 1h, 2h, 4h, 8h, 24h, 48h after injection), respectively.

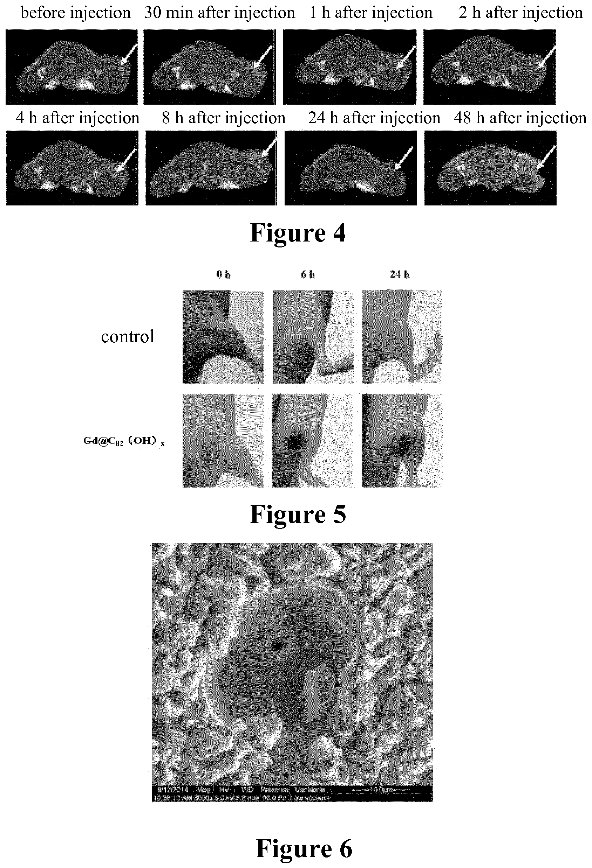

What are showed in Figure 4 are a series of figures of magnetic resonance imaging and state change of tumors after the radiofrequency pulses were applied to the water-soluble Gd@C₈₂ monocrystalline nanoparticles under a magnetic field (wherein the portions indicated by yellow arrows are tumors). It can be seen form the figures that significant changes occur in the tumor tissues at 4 h to 8 h after injection, the tumor tissues gradually necrose and blacken, the magnetic resonance imaging is low signal, and with the time, a wide range of tumor tissues are gradually cleared up.

Figure 5 is a series of photos of the changes of tumor tissues within 24 h after treatment with the methods of the present invention. It can be obviously observed that at 6 h after treatment the significant necrosis and blackening phenomena occur at tumor sites, and after 24 h the tumor has hollow interior and escharosis exterior, and most tumor tissues are cleared up by the body. However, normal biological tissues and organs such as liver, spleen, and kidney etc. remain intact. The imaging and anatomical results of animal experiments on other organs of tumor-bearing mice show that metallofullerene nanomaterials do not cause significant toxic and side effects on other organs, and can normally metabolize in the body.

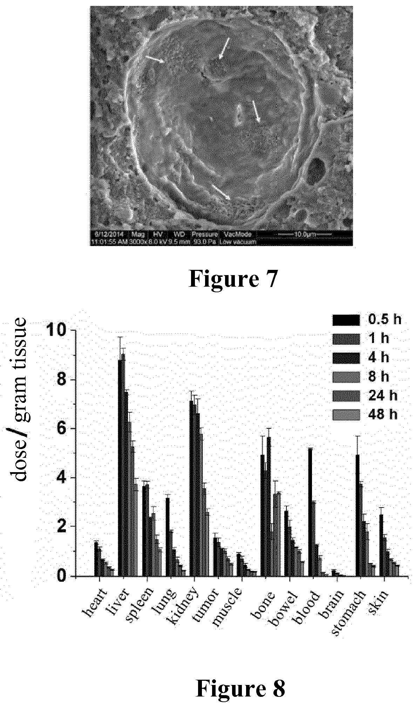

Figure 6 and Figure 7 show photos of environmental scanning electron microscope of tumor vessels after the radiofrequency effects without the treatments of water-soluble Gd@C₈₂ monocrystalline nanoparticles or with the treatments of water-soluble Gd@C₈₂ monocrystalline nanoparticles for two days, respectively. It can be observed from Figure 7 that after the effects of water-soluble Gd@C₈₂ monocrystalline nanoparticles and radiofrequency, a wide range of necrosis and exfoliation of endothelium cells occur in the tumor vessels , to expose the vessel basilemma, which illustrates that the impact force for the explosion of water-soluble Gd@C₈₂ monocrystalline nanoparticles under the role of radiofrequency can quickly and efficiently destroy tumor vessels, finally "starve" the tumor tissues, and achieve the effect of efficient and quick treatment.

The metal ions contained in metallofullerene showed in Figure 8 are protected by fullerene shell, and will not leak within the organism. ¹³¹I labeled experiments prove that they can normally metabolize within the organism, most of metallofullerenes can be metabolized out of the body after 48 hours, and the residual metallofullerenes in the body are lower than the limit of detection after 7 days, so they can not be retained in the body and not bring long-term toxic and side effects.

### Industrial Application

The method for the treatment of tumors provided by the present invention has a therapeutic effect on many tumors, without performing a minimally invasive surgery, and effectively avoids the metastasis of tumors by cutting off the tumor vessels, so it is a broad-spectrum, quick, safe, and noninvasive efficient method for the treatment of tumors.

## Claims

1. Use of metallofullerene monocrystalline nanoparticles for preparation of tumor vascular disrupting agents:
wherein, the metallofullerene monocrystalline nanoparticles are water-soluble metallofullerene monocrystalline nanoparticles with negative charges on their surfaces; the particle sizes of the monocrystalline nanoparticles range from 50 to 250 nanometers; the nanoparticles have rigidity, making them get stuck when they are passing through pores of vessel walls of the tumors; moreover, the nanomaterials are able to absorb outside radiation energy, transform it into heat energy, and accumulate the heat energy simultaneously, making the volumes of metallofullerene monocrystalline nanoparticles rapidly expand due to phase transition, thereby to cause changes in the morphologies, structures or functions of endothelium cells of tumor vessels.

2. The use according to Claim 1, **characterized in that**: metallofullerene in the metallofullerene monocrystalline nanoparticles is selected from at least one Of M@C₂ₙ, M₂@C₂ₙ, MA@C₂ₙ, M₃N@C₂ₙ, M₂C₂@C₂ₙ, M₂S@C₂ₙ, M₂O@C₂ₙ and MₓA₃₋ₓN@C₂ₙ; wherein M and A both represent metal elements, and M and A are both selected from any one of Sc, Y and lanthanide metal elements, 30 ≤ n ≤ 60; 0 ≤ x ≤ 3.

3. The use according to Claim 1 or Claim 2, **characterized in that**: the energy source providing the outside radiation energy is selected from at least one of radiofrequency, microwave, infrared light, visible light, laser, x-ray and alternating magnetic field.

4. The use according to any one of Claims 1-3, **characterized in that**: the metallofullerene monocrystalline nanoparticles are monocrystalline nanoparticles modified with hydroxyl groups, such as Gd@C₈₂(OH)ₓ; monocrystalline nanoparticles simultaneously modified with hydroxyl and amino groups, such as Gd@C₈₂(OH)ₓ(NH₂)_{y}; C60-based metallofullerene monocrystalline nanoparticles, such as Gd@C₆₀(OH)ₓ, Gd@C₆₀(COOH)ₓ etc.; or other metallofullerene monocrystalline nanoparticles such as Gd₃N@C₈₀(OH)ₓ(NH₂)_{y}, Gd@C₈₂(OH)ₓ(COOH)_{y}, and Lu₃N@C₈₀(OH)ₓ(NH₂)_{y} etc., the x all represent an integer between 10-30; the y all represent an integer between 0-20.

5. A drug kit for treating tumors, which consists of the metallofullerene monocrystalline nanoparticles of any one of Claim 1-4 and devices providing a radiation energy source matched with metallofullerene in the metallofullerene monocrystalline nanoparticles.

6. The drug kit according to Claim 5, **characterized in that**: the metallofullerene in the nanomaterials is nonparamagnetic metallofullerene, and the energy source matched therewith is a pulses laser; the metallofullerene in the nanomaterials is paramagnetic metallofullerene, and the energy source matched therewith is a radiofrequency.

7. A therapeutic method of tumors, including the following steps:
1) Administrating effective dose of metallofullerene monocrystalline nanoparticles to a tumor-bearing organism in need of treatment via injection; and
2) Irradiating tumor sites of the tumor-bearing organism using the radiation energy source matched with the metallofullerene monocrystalline nanoparticles.

8. The therapeutic method according to Claim 7, **characterized in that**: the organism refers to a mammal including a human being; and injection manner is intravenous injection.

9. The use according to any one of Claims 1-4, the drug kit according to any one of Claims 5-6, and the therapeutic method according to any one of Claims 7-8, **characterized in that**: the tumors are solid tumors.

10. The use, drug kit or therapeutic method according to Claim 9, **characterized in that**: the solid tumors include liver cancer, lung cancer, colorectal cancer, renal cancer, pancreatic cancer, bone cancer, breast cancer, ovarian cancer, prostate cancer, esophagus cancer, gastric cancer, oral cancer, nasal cancer, laryngeal cancer, liver cancer, bile duct cancer, cervical cancer, uterus cancer, testicular cancer, meningioma, skin cancer, melanoma, and sarcoma.
